# EUROPEAN PATENT SPECIFICATION

(11) **EP 3 029 156 B1**
(45) Date of publication and mention of the grant of the patent: **07.11.2018**
(21) Application number: 16150622.5
(22) Date of filing: 03.09.2010
(51) Int. Cl.: C12Q 1/6883

(54) **METHOD FOR THE DIAGNOSIS AND/OR PROGNOSIS OF ACUTE RENAL DAMAGE**
VERFAHREN ZUR DIAGNOSE UND/ODER PROGNOSE AKUTER NIERENSCHÄDEN
PROCÉDÉ POUR LE DIAGNOSTIC ET/OU LE PRONOSTIC DE LÉSION RÉNALE AIGUË

(30) Priority: 04.09.2009 ES 200901825
(43) Date of publication of application: 08.06.2016
(62) Divisional of application: 10813383.6
(73) Proprietor: Fundación Para La Investigación Biomédica Del Hospital Universitario Ramón Y Cajal, 28034 Madrid (ES)
(72) Inventor: GARCIA BERMEJO, Maria Laura, 28034 Madrid (ES); AGUADO FRAILE, Elia, 28034 Madrid (ES); LIAÑO GARCIA, Fernando, 28034 Madrid (ES); SAENZ MORALES, David, 28034 Madrid (ES)
(74) Representative: Hoffmann Eitle

(56) References cited:
- US-A1- 2005 266 418
- DANY ANGLICHEAU ET AL: "MicroRNA expression profiles predictive of human renal allograft status", PROCEEDINGS OF THE NATIONAL ACADEMY OF SCIENCES, NATIONAL ACADEMY OF SCIENCES, US, vol. 106, no. 13, 1 January 2009 (2009-01-01), pages 5330-5335, XP008152288, ISSN: 0027-8424, DOI: 10.1073/PNAS.0813121106 [retrieved on 2009-03-16]
- R. A. ZAGER ET AL: "Uremia impacts renal inflammatory cytokine gene expression in the setting of experimental acute kidney injury", AJP: RENAL PHYSIOLOGY, vol. 297, no. 4, 5 August 2009 (2009-08-05), pages F961-F970, XP055050776, ISSN: 0363-6127, DOI: 10.1152/ajprenal.00381.2009
- SUI ET AL: "Microarray analysis of MicroRNA expression in acute rejection after renal transplantation", TRANSPLANT IMMUNOLOGY, ELSEVIER, NL, vol. 19, no. 1, 5 February 2008 (2008-02-05), pages 81-85, XP022533984, ISSN: 0966-3274, DOI: 10.1016/J.TRIM.2008.01.007
- WEI LI ET AL: "MicroRNA detection by microarray", ANALYTICAL AND BIOANALYTICAL CHEMISTRY, SPRINGER, BERLIN, DE, vol. 394, no. 4, 9 January 2009 (2009-01-09), pages 1117-1124, XP019702594, ISSN: 1618-2650

## Description

### FIELD OF THE INVENTION

The present invention generally falls within the field of biomedicine and particularly relates to a method for the diagnosis and/or prognosis of acute renal damage.

### BACKGROUND OF THE INVENTION

In kidney transplant, acute tubular necrosis (ATN) is the main cause of delay in post-transplant graft function. In addition, ATN contributes to an increased incidence of acute rejection, the development of chronic rejection and decreased graft survival (Pannu *et al.,* 15 2008). The increase in the demand for organs in recent years involves the use of organs from sub-optimal donors, including non-heart beating donors and aging donors, which significantly increases the percentage of developing post-transplant ATN, morbidity of the graft and the delay in its functional recovery. This pushes up the total economic cost of a kidney transplant for public health systems. Note that the latest statistics of the National Transplant Organization (NTO) indicate that about 2,200 kidney transplants/year were performed in Spain and there are more than 4,000 patients still on waiting list (Dominguez-Gil and Pascual. 2008). In addition, ATN is the most common morphological manifestation of acute renal failure (ARF), including ARM resulting from ischemia (Kellum *et al.,* 2008). ARF represents one of the most serious problems among kidney diseases in the developed world due to its high mortality, around 50%. Around 30% of all ARF episodes occur in patients admitted to ICUs as a result of multiple organ failure. In the latter context, mortality rises to 80% (Chertow *et al.,* 2005). The development of ARF is also one of the most common complications after cardiac intervention, around 30,000/year of which are carried out in Spain, and more than one 1% of them in our Hospital. Practically all the patients that are operated on develop a certain degree of ARF (Yates and Stafford-Schmit, 2006). Long-term progress of patients depends on the severity of this post-operative ARF, resulting in a mortality approaching 60% in those cases requiring dialysis after the cardiac intervention (Takar *et al.,* 2005; Candela-Toha *et al.,* 2008). Both kidney transplant and cardiac surgery are two "quasi" experimental study situations for human ATN, since the moment and duration of the ischemic stimulus is known and they can also be monitored. All these statistics on morbidity and mortality have not changed significantly in recent decades and so far, there has been no effective therapy for the prevention and/or reduction of ATN in all these situations. The lack of kidney damage markers that are more accurate than the determination of serum creatinine and urea, which has been used up until now, has greatly contributed to this. These classic markers do not directly reflect the cell damage or do they show the compartment of the kidney tissue (tubule or endothelium) in which such damage is taking place. They are only indicative parameters of disturbed renal function as a result of the damage (Vaidya *et al.,* 2008). In fact, it is possible that patients with subclinical renal damage are not identified as such because a significant disturbance in serum creatinine and urea levels has not taken place. Thus, in recent years numerous studies have been developed which have tried to identify and validate new ARF markers, such as NGAL, IL18, KIM, Cystatin C, VEGF or CXCL10, which seem to work as good markers in child populations without significant added pathologies but not in an adult population (Vaidya *et al.,* 2008).

Renal ischemia, hypovolemia and toxic agents are the most frequent causes of development of ATN. The reduction in blood flow, and as a result tissue hypoxia, result in damage at the level of the proximal tubular epithelium, cause a rapid decrease in glomerular filtration, disturb vascular permeability and trigger an inflammatory response that amplifies tissue damage (Thurman *et al.,* 2007). The degree and extent of the ischemic damage are dependent on the severity and duration of ischemia. In sublethal ischemia, the detachment of proximal epithelial cells, many of which are viable, into the tubular lumen can be seen. In more prolonged ischemia, persistent tissue hypoxia and the inflammatory response, *inter alia,* increase epithelial and vascular damage, with cell death in the cortical-medullary area of the kidney. In addition, the vascular compartment is also damaged after ischemia. In fact, endothelial damage significantly contributes to acute renal damage and also to the maintenance thereof over time. Early disturbances in peritubular flow during ischemia and early reperfusion are associated with loss of endothelial morphology and function contributing to the loss of barrier function, inflammation and procoagulant activity. In the mid- to long-term, loss of microvascular density, favoring progression of chronic kidney damage as a direct result of initial ischemia, has been described (Basile 2007). To resolve ATN, mechanisms that facilitate tissue repair are started up, .i.e., cell division and differentiation from undamaged epithelial tubular cells. In recent years, several studies have demonstrated that not only undamaged epithelial cells themselves that are de-differentiated and proliferate, but also renal pluripotent cells and even extrarenal pluripotent cells such as those from bone marrow can contribute to the repair of tubular damage after ischemia (Lin 2008). However, the contribution of stem cells to the repair of ischemic damage is questioned, although it is accepted that fundamentally undamaged proximal tubular cells and parenchymal revascularization would contribute to such repair. In this latter process, it has been proposed that endothelial progenitor cells mobilized after ischemia would also participate (Becherucci *et al.,* 2009).

miRNAs are small-sized (22-25 nucleotides), endogenously encoded RNAs able to recognize messenger RNAs and thus negatively regulate protein expression, within RNA-induced silencing complexes (RISC) by total or partial complementarity with its target mRNA (Chang and Mendell, 2007). In humans more than 700 have already been cloned, and bioinformatic predictions indicate that all of them can control expression of more than 30% of total proteins (Filipowicz *et al.,* 2008). Most are transcribed by RNA Pol II from individual genes or from polycistronic transcripts for several of them at the same time. They are generated as longer pre- miRs processed in the nucleus by a Ribonuclease III (Drosha), go to the cytoplasm via Exportin- 5- and Ran-GTP-dependent mechanisms and there they are finally processed by another Ribonuclease III (Dicer) to their mature form (Rana 2007). Their function is essential in a wide range of processes, including embryonic development, response to stress or strict regulation of physiological processes and, therefore, maintenance of homeostasis in organisms. It is important to highlight that the miRNA expression profile is cell type-specific and can change depending on the stimulus, such that the particular cell context of one and the same miRNA will determine its function in a specific cell type (Bartel 2009). For this reason, deregulation of certain miRNAs has been pointed out among the mechanisms responsible for the development of pathologies such as cancer (Bartels and Tsongalis, 2009), autoimmunity (Sonkoly and Pivarcsi 2008), diabetes (Zhou *et al.,* 2008) or vascular pathologies (Urbich *et al.,* 2008) and are being constituted as precise biomarkers of the progress of many of them. Very recently it has been demonstrated that miRNAs are furthermore key regulators in rapid and precise cellular response to any type of stimulus, including the lack of nutrients or hypoxia (Ivan *et al.,* 2008). In addition, it has also been demonstrated that these miRNAs along with mRNAs can be secreted or exchanged by cells in the form of microparticles (platelet-derived microvesicles; tumor cell exosomes; neutrophil ectosomes (Valadi *et al.,* 2007). They could thus be detected in body fluids such as blood, urine or pleural fluid. In fact, it is estimated that the peripheral blood of healthy individuals can contain a concentration between 5-50 mg/ml of microparticles, which would increase in the case of patients with various pathologies (Hunter *et al.,* 2008). This would allow performing a very reliable monitoring of the progress of these pathologies using samples obtained by minimally invasive methods (blood extraction and urine collection) (Gilad 2008). In urine, the detected miRNAs, among which miR-127 is included, have shown great stability, even under very aggressive conditions (Melkonyan *et al.,* 2008). Given that miRNA deregulation can cause various pathologies, they are beginning to be regarded as new targets of therapeutic action. In fact, tools have been developed to modulate the expression thereof: pre-miRs for their over-expression and antagomiRs (anti-miRs) for their inhibition, with very hopeful results in various experimental models *in vitro* and *in vivo* (Krutzfeld *et al.,* 2006; Care *et al.,* 2007; Van Rooij *et al.,* 2008), although the validity thereof as a therapeutic strategy in humans has yet to be determined.

Regarding the role of miRNAs in response to ischemia, the expression thereof in focal cerebral ischemia in rats has been determined, establishing an association between miR-145 expression and brain damage (Dharap *et al.,* 2009). In cardiac ischemia in humans, miR-100 and miR-133 seem to participate in the mechanism of cardiac damage (Sucharov C, *et al.,2008).* In hepatic ischemia also in humans, miR-223 has been established as a mediator of damage (Yu *et al.,* 2008). In contrast, miR-126 and miR-210 have been described as fundamental promoters of angiogenesis, neovascularization and tissue repair in response to various stimuli, including hypoxia (Suarez and Sessa, 2009; Fasanaro *et al.,* 2008; van Solingen *et al.,* 2008). Up until now, miRNAs modulated in renal I/R have not been described in the literature, but experts have begun to speculate about their potential as biomarkers in renal pathologies, including those involving disturbances in blood pressure regulation (Liang M *et al.,2008).* In another context, some miRNAs related to immune rejection in kidney transplant have been identified (Sui *et al.,* 2008).

The preceding justifies the need to identify and validate new biomarkers of kidney damage progression that are more accurate and indicative of what tissue compartment is being damaged and/or is recovering and to what extent this is taking place, the determination of which is furthermore rapid and easy and does not require a patient biopsy.

### DESCRIPTION OF THE INVENTION

Therefore in a first aspect, the present invention provides a method for the diagnosis and/or prognosis of acute renal damage which comprises analyzing a sample obtained from a patient to determine the expression level of at least miR-127, and compare said expression level with a control value, where the disturbance of said level is indicative of renal damage.

In a more particular aspect of the present invention, the sample from the patient to be analyzed is blood. In another particular aspect of the present invention, the sample is serum. In another aspect of the present disclosure, the sample is urine.

In a more particular aspect of the present invention, the diagnosis and/or prognosis of acute renal damage is carried out by determining the expression level of miR-127 alone or in combination with at least one microRNA selected from miR-126, miR-210 and miR-101.

In a more particular aspect of the present invention, the decrease in the expression level of serum miR-127 with respect to the control value is indicative of acute renal damage.

In a more particular aspect of the present invention, the increase in the expression level of urinary miR-127 with respect to the control value is indicative of acute renal damage.

In a more particular aspect of the present disclosure, the diagnosis and/or prognosis of acute renal damage is carried out by determining the expression level of miR-126 alone or in combination with at least one microRNA selected from miR-127, miR-210 and miR-101.

In a more particular aspect of the present disclosure, the decrease in the expression level of serum miR-126 with respect to the control value is indicative of acute renal damage.

In a more particular aspect of the present disclosure, the diagnosis and/or prognosis of acute renal damage is carried out by determining the expression level of miR-210 alone or in combination with at least one microRNA selected from miR-127, miR-126 and miR-101.

In a more particular aspect of the present disclosure, the increase in the expression level of serum miR-210 with respect to the control value is indicative of acute renal damage.

In a more particular aspect of the present disclosure, the decrease in the expression level of urinary miR-210 with respect to the control value is indicative of acute renal damage.

In a more particular aspect of the present disclosure, the diagnosis and/or prognosis of acute renal damage is carried out by determining the expression level of miR-101 alone or in combination with at least one microRNA selected from miR-127, miR-126 and miR-210.

In a more particular aspect of the present disclosure, the increase in the expression level of serum miR-101 with respect to the control value is indicative of acute renal damage.

In the present invention, "acute renal damage" refers to kidney damage with an ischemic etiology that is either primary or secondary, as is the case of kidney damage due to toxic agents or radiocontrast media, and in any case excluding chronic kidney damage.

In a more particular aspect of the present invention, microRNA expression is determined by PCR. In a more particular aspect, microRNA expression is determined by quantitative PCR. In a more particular aspect, microRNA expression is determined by multiplex PCR.

In another more particular aspect of the present invention, microRNA expression is determined by total RNA microarrays.

In a second aspect, the present disclosure relates to a kit for the diagnosis and/or prognosis of acute renal damage comprising the probes and primers needed for carrying out the method of the present disclosure.

In a more particular aspect of the present disclosure, the kit comprises the probes and primers needed for determining the expression level of at least one microRNA selected from miR-127, miR-126, miR-210 and miR-101.

In another more particular aspect of the present disclosure, the kit comprises an RNA microarray.

### DESCRIPTION OF THE DRAWINGS

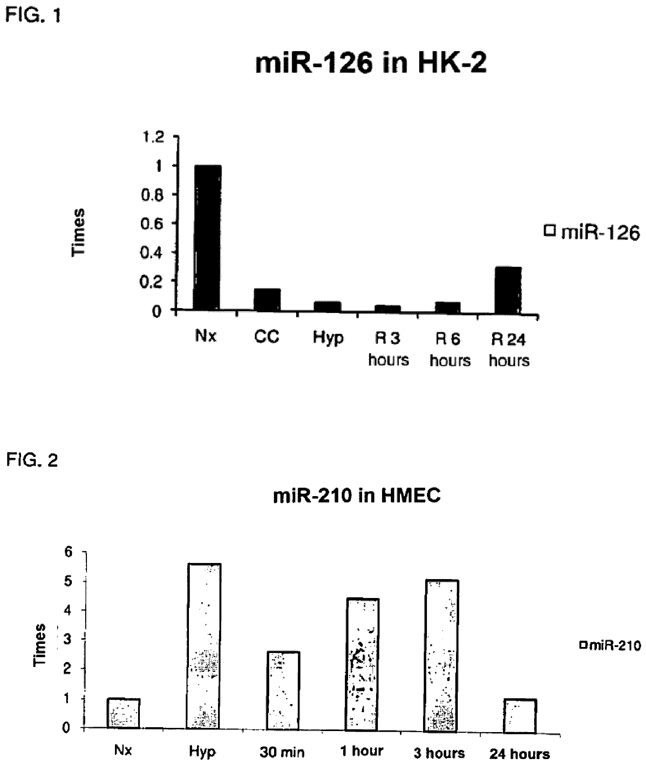

Figure 1 shows miR-126 expression in HK-2 human proximal tubular cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction for 6 h (1% oxygen in nutrient-free medium); R-3, R-6, R-24 h: cells that after 6 h of hypoxia are again under normal conditions of oxygen and nutrient availability.
Figure 2 shows miR-210 expression in HK-2 human proximal tubular cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction (1% oxygen in nutrient-free medium); R-3, R-6, R- 24 h: cells that are again under normal conditions of oxygen and nutrient availability.

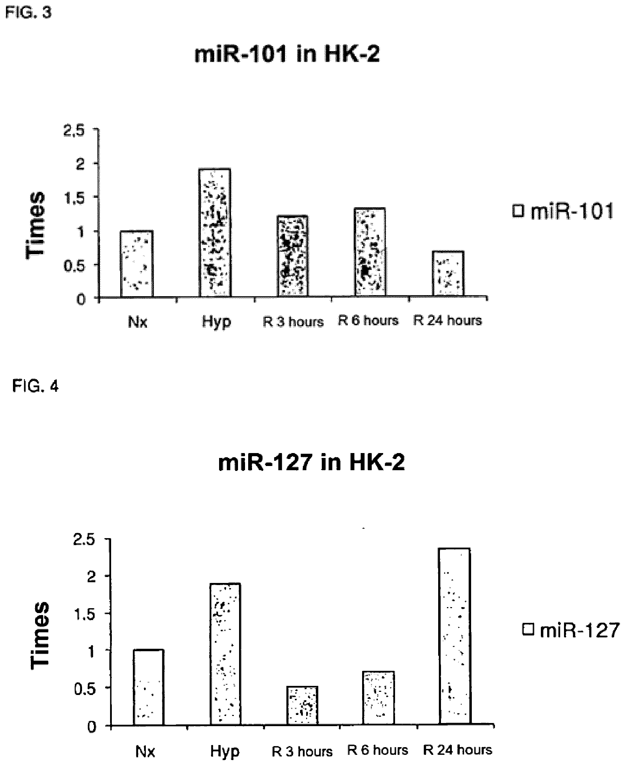

Figure 3 shows miR-101 expression in HK-2 human proximal tubular cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction (1% oxygen in nutrient-free medium); R-3, R-6, R- 24 h: cells that are again under normal conditions of oxygen and nutrient availability.
Figure 4 shows miR-127 expression in HK-2 human proximal tubular cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction (1% oxygen in nutrient-free medium); R-3, R-6, R- 24 h: cells that are again under normal conditions of oxygen and nutrient availability.

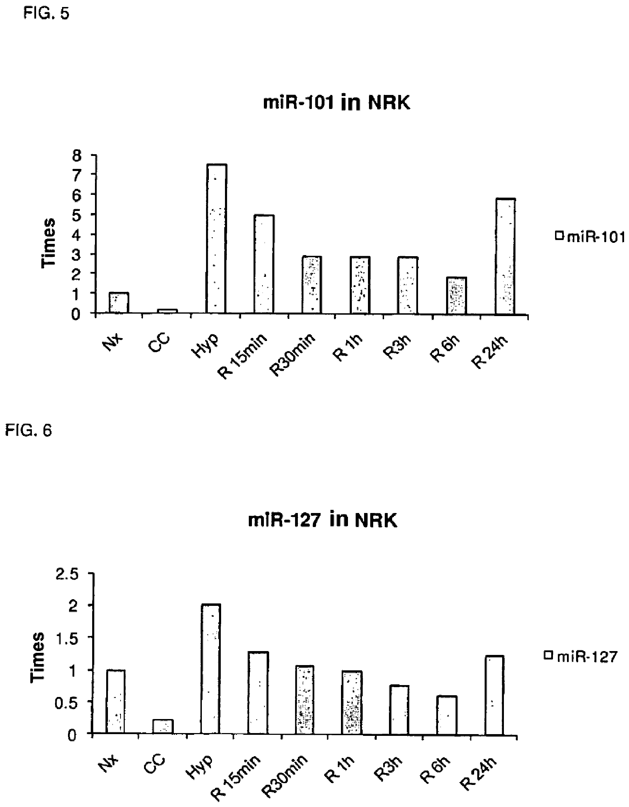

Figure 5 shows miR-101 expression in NRK-52E rat proximal tubular cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction (1% oxygen in nutrient-free medium); R-15 min, R- 30 min, R-1 h, R-3 h, R-6 h, R-24 h: cells that are again under normal conditions of oxygen and nutrient availability.
Figure 6 shows miR-127 expression in NRK-52E rat proximal tubular cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction (1% oxygen in nutrient-free medium); R-15 min, R- 30 min, R-1 h, R-3 h, R-6 h, R-24 h: cells that are again under normal conditions of oxygen and nutrient availability.

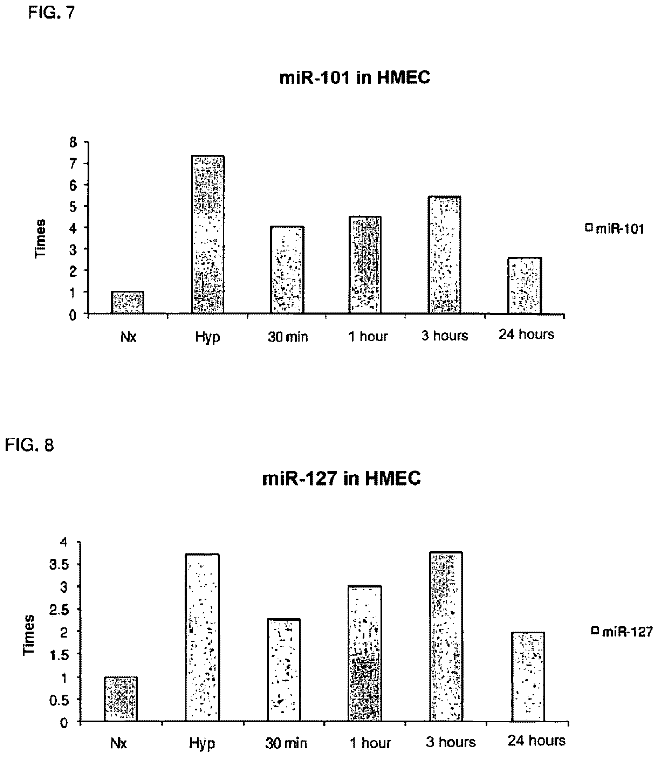

Figure 7 shows miR-101 expression in HMEC human endothelial cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction (1% oxygen in nutrient-free medium); R-15 min, R- 30 min, R-1 h, R-3 h, R-24 h: cells that are again under normal conditions of oxygen and nutrient availability.
Figure 8 shows miR-127 expression in HMEC human endothelial cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction (1% oxygen in nutrient-free medium); R-15 min, R- 30 min, R-1 h, R-3 h, R-24 h: cells that are again under normal conditions of oxygen and nutrient availability.

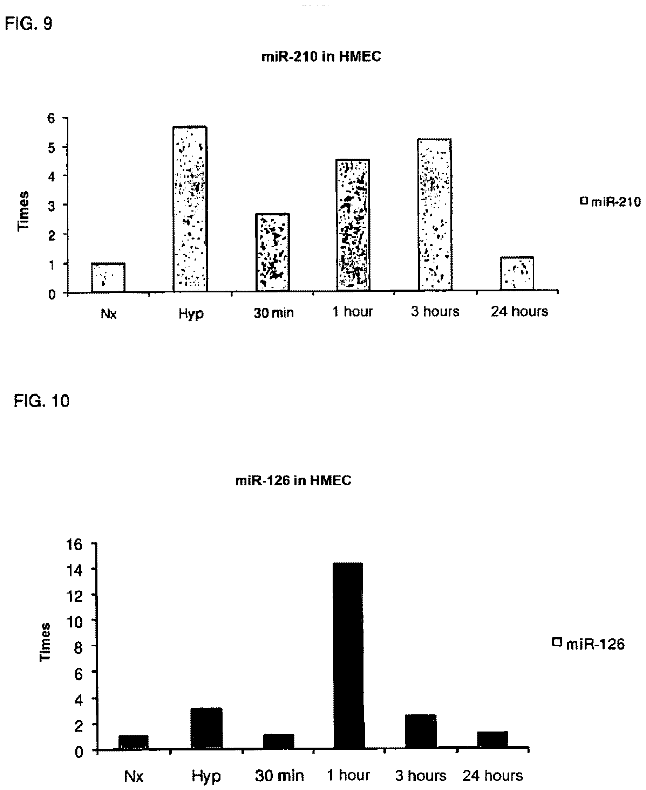

Figure 9 shows miR-210 expression in HMEC human endothelial cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction (1% oxygen in nutrient-free medium); R-15 min, R-30 min, R-1 h, R-3 h, R-24 h: cells that are again under normal conditions of oxygen and nutrient availability.
Figure 10 shows miR-126 expression in HMEC human endothelial cells subjected to the hypoxia/reoxygenation protocol. NX: cells under normal conditions with regard to oxygen availability (normoxia, 21%) and nutrient availability (10% FBS complete medium) CC: control cells that have suffered nutrient restriction (they are in nutrient-free medium). Hyp: cells that have suffered nutrient and oxygen restriction (1% oxygen in nutrient-free medium); R-15 min, R- 30 min, R-1 h, R-3 h, R-24 h: cells that are again under normal conditions of oxygen and nutrient availability.

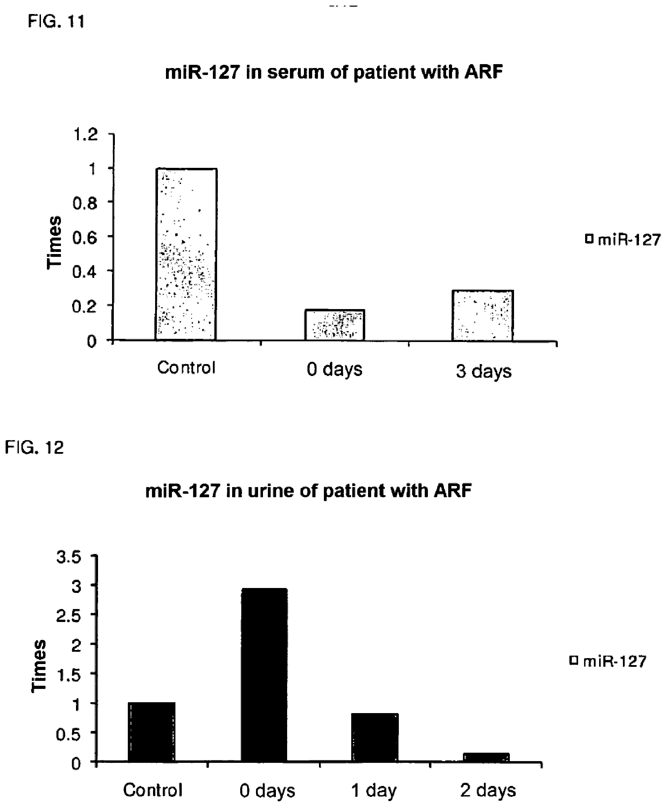

Figure 11 shows serum miR-127 expression in patients diagnosed with acute renal failure (ARF) of ischemic etiology. Control: miRNA expression in healthy control equaled to 1. Expression data in the patient is relativized to this data. 0 h and 3 days: times at which a sample has been taken from the patient, upon admittance for ARF (0 h) and later during progression thereof (3 days).
Figure 12 shows urinary miR-127 expression in patients diagnosed with acute renal failure (ARF) of ischemic etiology. Control: miRNA expression in healthy control equaled to 1. Expression data in the patient is relativized to this data. 0 h and 3 days: times at which a sample has been taken from the patient, upon admittance for ARF (0 h) and later during progression thereof (3 days).

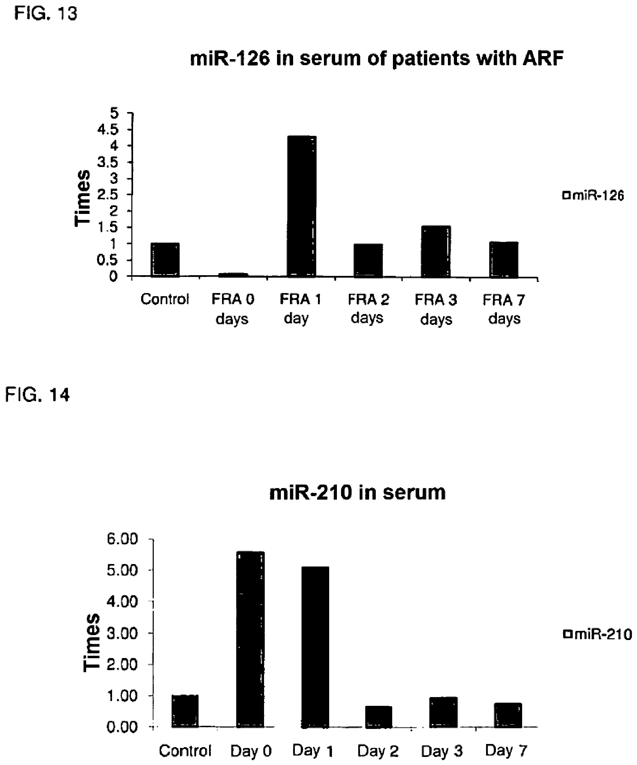

Figure 13 shows serum miR-126 expression in patients diagnosed with acute renal failure (ARF) of ischemic etiology. Control: miRNA expression in healthy control equaled to 1. Expression data in the patient is relativized to this data. 0 h and 3 days: times at which a sample has been taken from the patient, upon admittance for ARF (0 h) and later during progression thereof (3 days).
Figure 14 shows serum miR-210 expression in patients diagnosed with acute renal failure (ARF) of ischemic etiology. Control: miRNA expression in healthy control equaled to 1. Expression data in the patient is relativized to this data. Day 0, Day 1, Day 2, Day 3, Day 7: times at which a sample has been taken from the patient, upon admittance for ARF (day 0) and later during progression thereof.

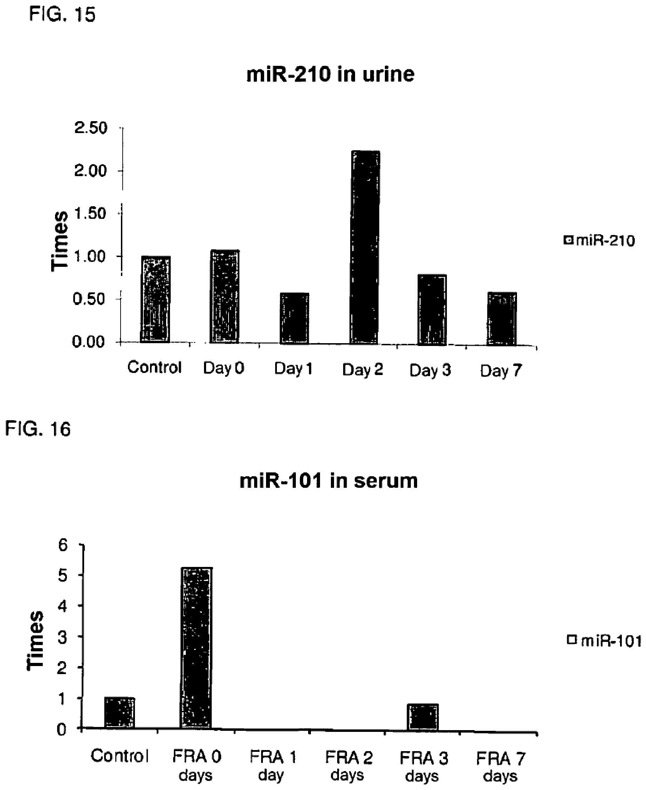

Figure 15 shows urinary miR-210 expression in patients diagnosed with acute renal failure (ARF) of ischemic etiology. Control: miRNA expression in healthy control equaled to 1. Expression data in the patient is relativized to this data. Day 0, Day 1, Day 2, Day 3, Day 7: times at which a sample has been taken from the patient, upon admittance for ARF (day 0) and later during progression thereof.
Figure 16 shows serum miR-101 expression in patients diagnosed with acute renal failure (ARF) of ischemic etiology. Control: microRNA expression in healthy control equaled to 1. Expression data in the patient is relativized to this data. 0 days, 1 day, 2 days, 3 days, 7 days: times at which a sample has been taken from the patient, upon admittance for ARF (0 d) and later during progression thereof.

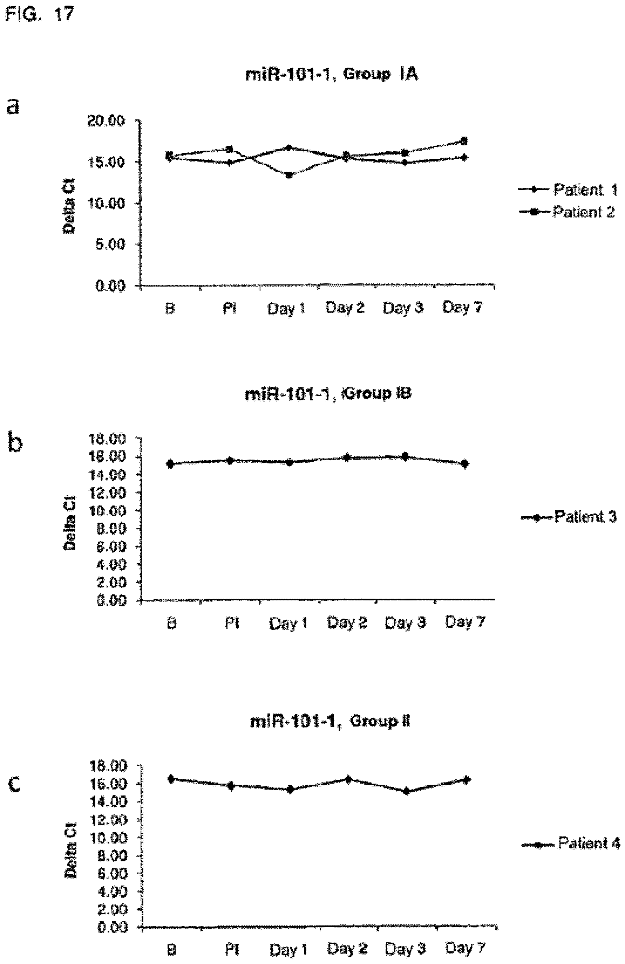

Figure 17 shows miR-101 expression in serum samples a: in patients belonging to group IA, b: in patients belonging to group IB, and c: in patients belonging to group II.

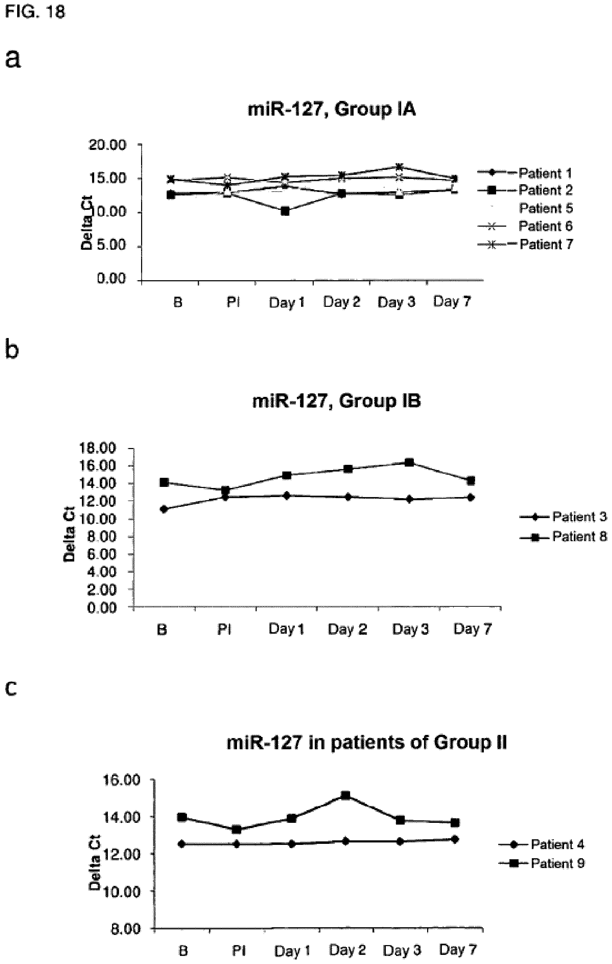

Figure 18 shows miR-127 expression in serum samples a: in patients belonging to group IA, b: in patients belonging to group IB, and c: in patients belonging to group II.

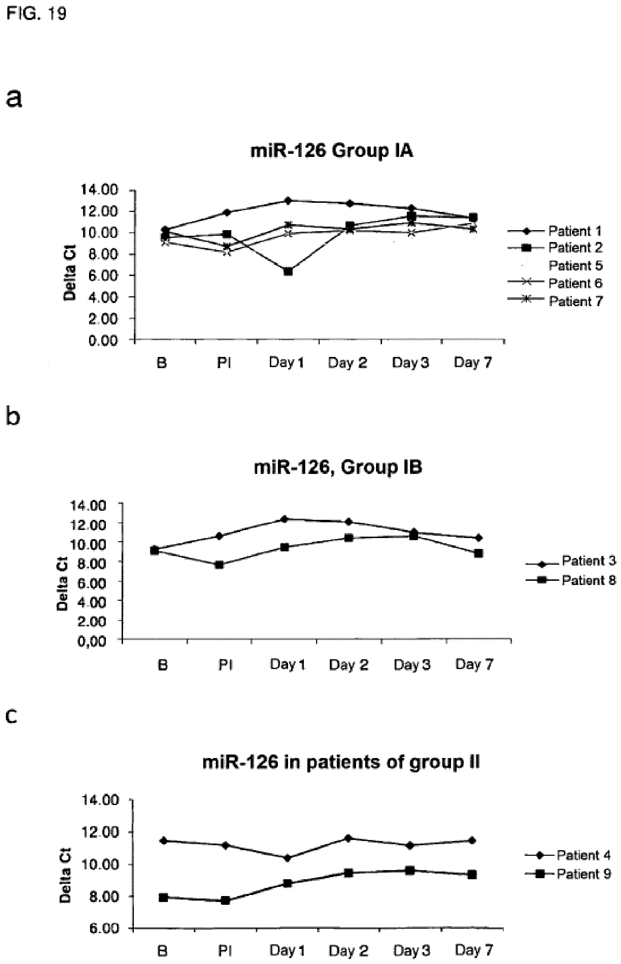

Figure 19 shows miR-126 expression in serum samples a: in patients belonging to group IA, b: in patients belonging to group IB, and c: in patients belonging to group II.

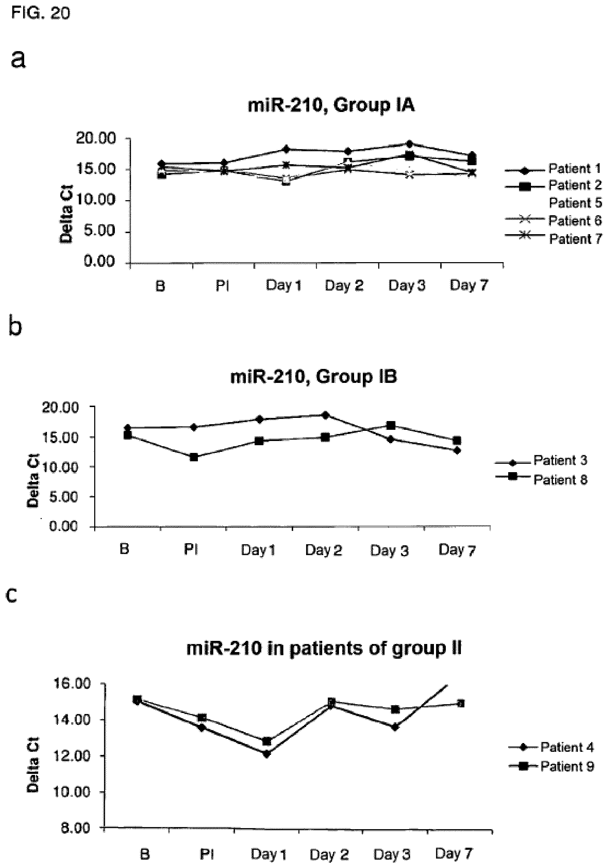

Figure 20 shows miR-210 expression in serum samples a: in patients belonging to group IA, b: in patients belonging to group IB, and c: in patients belonging to group II.

### DETAILED DESCRIPTION OF THE INVENTION

It was identified by using arrays that microRNAs: miR-127, miR-126, miR-210 and miR-101 in an H/R model mimicking I/R were differentially expressed such that each of these microRNAs, alone or in combination, serve as biomarkers of kidney damage.

### EXAMPLE 1: miRNA expression in cell lines subjected to hypoxia/reoxyqenation.

The following cells (HK2: human proximal tubular cells, NRK-52E: rat proximal tubular cells, and HMEC: human microvascular endothelial cells) were cultured in suitable media containing specific serum, antibiotics and growth factors. They were kept at 37°C, under a humid atmosphere and with 5% CO₂.

The cell lines described above were subjected to a hypoxia/reoxygenation protocol. That is, cell lines are subjected to changes in oxygen tensions and nutrient availability. For that purpose, two different incubators were used: hypoxia in a sealed incubator at 37°C, perfused with a mixture of 5% CO₂, 1% O₂, 94% N₂; reoxygenation in a standard incubator at 37°C, with 5%
CO₂. The cells were grown to confluence and deprived of serum 24 hours before hypoxia. During hypoxia, they were kept in serum-free minimal medium (HBSS), with a low concentration of glucose or derivatives. During reoxygenation, complete medium was used (Saenz-Morales *et al.,* 2006). The hypoxia time for all samples was 6 h, and reoxygenation times were variable (15 min-72 h). All the *in vitro* experiments were repeated at least 3 times.

Then expression of different microRNAs was determined in the cell lines by PCR, for which purpose and after extraction of total RNA from the samples of fluids (serum or urine) and for titration, 50 nanograms of each one of them were used for the reverse transcription (RT) reaction in 15 microliters. Special commercial primers (stem loop primers) were used for this step. These primers were specific to each miRNA. After RT, quantitative amplification reaction (qPCR) was performed. In this reaction, carried out in a total volume of 10 microliters, 1 microliter of the total RT reaction and primers specific for each miRNA, as well as a Taqman probe with fluorescence trapping agents, were used. All reagents, both primers and reaction mixtures with enzymes and nucleotides for RT and PCR that were used were from Applied Biosystems. The results were as follows:

As shown in Figure 1, the expression levels of miR-126 were heavily dependent on the nutrient and oxygen availability since the restriction of both very significantly decreased the expression thereof with respect to the normality condition. microRNA expression was recovered in the reoxygenation time, in which cells again had oxygen and nutrients available to them. Therefore, the decrease in miR-126 expression indicated lack of nutrients and oxygen in proximal tubular cells, which is indicative of renal ischemia. In addition, and given that at 3 h of reoxygenation significant damage to the epithelium *in vitro* was recorded, low expression of this miRNA indicated damage of the proximal tubular epithelium after ischemia.

As shown in Figure 2, the expression levels of miR-210, like miR-126, were heavily dependent on the nutrient and oxygen availability, since the restriction of both very significantly decreased the expression thereof with respect to the normality condition. microRNA expression was recovered in the reoxygenation time, in which cells again had oxygen and nutrients available to them. The decrease in miR-210 indicated lack of nutrients and oxygen in the proximal tubular cells, which is indicative, as in the case of miR-126, of renal ischemia. In addition, and given that at 3 h of reoxygenation damage to the epithelium *in vitro* was recorded, the low miR-210 expression indicated damage of the proximal tubular epithelium after ischemia.

As shown in Figure 3, miR-101 expression increased under hypoxia condition compared to normoxia condition. Expression of this miRNA was gradually normalized again in reoxygenation, in which oxygen and nutrient availability was normalized again. The increase in expression of this miRNA in proximal cells during hypoxia condition was indicative of renal ischemia.

As shown in Figure 4, miR-127 expression increased under hypoxia condition compared to normoxia condition. Expression of this miRNA decreased significantly under reoxygenation, increasing again at 24 h of reoxygenation when the epithelial monolayer is recovering. Therefore, the increase in miR-127 expression in proximal cells during hypoxia condition was indicative of renal ischemia. The decrease in expression thereof early on under reoxygenation indicated ischemic damage and the subsequent increase thereof indicated endothelial recovery.

As shown in Figure 5, miR-101 expression rapidly normalized under reoxygenation, in which oxygen and nutrient availability was normalized again, even though it remained with higher expression levels than under normoxia condition in these rat cells. The increase in expression of this miRNA in proximal cells during hypoxia condition was indicative of renal ischemia. The new increase thereof at 24 h of reoxygenation indicated recovery of the epithelial monolayer.

As shown in Figure 6, and as occurred in Hk-2 (human tubular) cells, miR-127 expression increased under hypoxia condition, in which nutrient and oxygen availability is restricted, compared to normoxia condition. Expression of this miRNA rapidly normalized under reoxygenation, in which oxygen and nutrients were available again. The increase in expression of this miRNA in proximal cells during hypoxia condition was indicative of renal ischemia.

As shown in Figure 7, and as occurred in proximal tubular cells, miR-101 expression increased under hypoxia condition compared to normoxia condition. Expression of this miRNA remained high under reoxygenation, in which oxygen and nutrient availability was normalized again, and began to be normalized at 24 h of reoxygenation. The increase in expression of this miRNA in endothelial cells during hypoxia condition was indicative of renal ischemia. High expression thereof under reoxygenation was associated with an endothelial activation (pro-inflammatory) state, which began to be normalized at 24 h.

As shown in Figure 8, and as occurred in proximal tubular cells, miR-127 expression increased under hypoxia condition, in which nutrient and oxygen availability was restricted, compared to normoxia condition. Expression of this miRNA remained high under reoxygenation, in which oxygen and nutrients were available, and began to be normalized at 24 h of reoxygenation. The increase in expression of this miRNA in endothelial cells during hypoxia condition was indicative of renal ischemia. High expression thereof under reoxygenation was associated with an endothelial activation (pro-inflammatory) state, which would begin to be normalized at 24 h.

As shown in Figure 9, and unlike what occurred with this miRNA in proximal tubular cells, miR- 210 expression increased discreetly under hypoxia condition, compared to normoxia condition.

Expression of this miRNA remained high under reoxygenation and expression abruptly decreased at 24 h of reoxygenation. The increase in expression of this miRNA in endothelial cells during hypoxia condition was indicative of renal ischemia. Like miR-127 and miR-101, expression thereof under reoxygenation was associated with an endothelial activation (pro- inflammatory) state, which began to be normalized at 24 h.

As shown in Figure 10, miR-126 expression increased discreetly under hypoxia condition, in which nutrient and oxygen availability is restricted, compared to normoxia condition. It increased very significantly at 1 h of reoxygenation, in which oxygen and nutrients were available. After that, expression of this miRNA was normalized. The increase in expression of this miRNA in endothelial cells during hypoxia condition was indicative of renal ischemia. Such a significant increase at 1 h of reoxygenation was unequivocally associated with a maximum endothelial activation (pro-inflammatory) state.

### EXAMPLE 2: miRNA expression in patients that have been diagnosed with acute renal failure.

The study with samples from patients was carried out in a prospective manner. After authorization by patients or their legal representatives by means of the pertinent informed consent and prior approval of the study by the Clinical Research Ethics Committee of our Hospital, blood and urine samples and renal biopsies were extracted in the case of transplant in the patient groups described below.

### Samples from kidney transplant patients

Samples from 50 transplant patients (patients with de novo kidney transplant and with different immunosuppression regimens) were analyzed, said patients being organized into two groups:
I. 25 patients with immediate graft function
II. 25 patients with delayed graft function

Blood and urine samples were taken on post renal transplant days 1, 7, 15, 30, and in them expression of the miRNAs to be studied was determined by qRT-PCR.

In the case of graft non-function, a biopsy was performed on the seventh day that was repeated every 7-10 days until the ATN phase was resolved. In case of suspected rejection and after confirmation by biopsy, these patients were excluded.

In the case of transplanted patients, the following information was collected and made available:
- Recipient characteristics: age, sex, and time on dialysis.
- Donor characteristics: type of donor (brain dead, non-heart beat), age, sex, need for vasoactive drugs and last creatinine.
- Graft characteristics: times of hot, cold ischemia and of anastomosis. HLA compatibility and immunosuppression.
- Graft function at 2, 4 and 12 weeks.

Blood samples were collected in 8 ml VACUETTE tubes (z serum sep clot activator), that were centrifuged at 2500 rpm for 10 minutes.

The serum separated by centrifugation was collected and aliquoted and stored in accordance with the Ramon and Cajal Hospital BioBank criteria (in anonymized tubes, with a unique code and at - 80°C).

Urine samples were collected in standard urine vials or extracted from the catheter bag, centrifuged at 2800 rpm for 10 minutes to remove sediments and other residues, and aliquoted and stored in accordance with the Ramon and Cajal Hospital BioBank criteria (in anonymized tubes, with a unique code and at -80°C).

Transfer of all these samples was requested by the BioBank by means of transfer agreements. A maximum of 500 microliters was requested since the technique was optimized for amplifying miRNAs in 100-200 microliter samples of both fluids, by quantitative PCR, for which purpose and after extraction of total RNA from the samples of fluids (serum or urine) and for titration, 50 nanograms of each one of them were used for the reverse transcription (RT) reaction in 15 microliters. Special commercial primers (stem loop primers) were used for this step. These primers were specific to each miRNA. After RT, quantitative amplification reaction (qPCR) was performed. In this reaction, carried out in a total volume of 10 microliters, 1 microliter of the total RT reaction and primers specific for each miRNA, as well as a Taqman probe with fluorescence trapping agents, were used. All reagents, both primers and reaction mixtures with enzymes and nucleotides for RT and PCR that were used were from Applied Biosystems.

Processing of the serum and urine samples from the patients prior to total RNA extraction was as follows: an aliquot of 100-200 microliters of serum or urine was digested with Proteinase K (0.65 milligrams/milliliter) incubating at 56°C, 1 h. After that, a first extraction with phenol/chloroform (5:1) was carried out and the aqueous phase was processed using the High
Pure miRNA isolation Kit (Roche), following the manufacturer's instructions.

### Samples from patients after cardiac surgery:

Samples from 50 patients were analyzed, said patients being organized into the following groups:
- IA: 10 adult patients operated on as scheduled with extracorporeal circulation (ECC) and with a low risk for developing ARF, i.e., patients with a score of 0 to 2 in the Thakar5 system or 0 to 1 in the simplified SRI6.
- IB: 10 pediatric patients with congenital heart disease operated on for the first time with ECC.
- II: 15 adult patients operated on as scheduled with ECC, with disturbed baseline renal function and scores >5 in the Thakar5 system or >3 in SRI6.
- III: 15 adult patients operated on as scheduled with ECC, with normal baseline renal function and with the same scores as in the previous section.

For each patient, the mentioned miRNAs were determined at the following times:
- Preoperative baseline
- At 2 h on admittance in ICU
- 24 h, 48 h and 72 h after surgery
- On day +7 (optional for groups IA and IB)

As shown in Figure 11, miR-127 expression was significantly decreased with respect to the healthy control. The decrease in expression of this miRNA in the serum from patients was an indicator of ischemic renal damage or ARF. This data correlates with the data shown in Figure 18.

As shown in Figure 12, and in correlation with the decrease in serum, miR-127 expression increased significantly with respect to the healthy control. The decrease in expression of this miRNA in the serum from patients and the corresponding increase in urine was an indicator of early diagnosis of ischemic renal damage or ARF.

As shown in Figure 13, expression levels of serum miR-126 decreased significantly at the time of onset of ischemic renal damage and expression thereof greatly increased at 24 h, to be subsequently normalized. This indicated that the decrease in expression of this miRNA in the serum from patients was an indicator of very early diagnosis of ischemic renal damage or ARF. Its increase at 24 h indicated endothelial activation after ischemia associated with a subsequent inflammatory reaction, as discussed in the in vitro model. This data correlates with the data shown in Figure 19.

As shown in Figure 14, serum miR-210 expression increased significantly at the time of onset of ischemic renal damage; it was maintained in the first 24 h, to be subsequently normalized. This data indicated that the increase in expression of this miRNA in the serum from patients was a very early diagnostic marker of ischemic renal damage or ARF.

As shown in Figure 15, urinary miR-210 expression increased significantly at 48 h, to be subsequently normalized. Taking into account that expression of this miRNA increased in HK-2 cells at times in which epithelial recovery in the experimental model began to be observed, this indicated that the increase in expression of this miRNA at 48 h in urine from patients was 15 indicative of the onset of recovery after ischemic renal damage. This data correlates with the data shown in Figure 20.

As shown in Figure 16, miR-101 expression increased very significantly and very early with respect to the healthy control, at the time of admittance, and no significant expression with respect to the healthy subject in the time of progression was detected. This indicated that an increase in expression of this miRNA in serum from patients was a very early diagnostic marker of ischemic renal damage. This data correlates with the data shown in Figure 17.

## Claims

1. A method for the diagnosis and/or prognosis of acute renal damage which comprises analyzing a sample from a patient selected from blood or serum, to determine the expression level of at least miR-127, and comparing said expression level with a control value, wherein the decrease in the expression level of serum miR-127 with respect to the control value is indicative of acute renal damage.

2. The method for the diagnosis and/or prognosis of acute renal damage according to claim 1, wherein it further comprises determining the expression level of miR-126, wherein the decrease in the expression level of miR-126 with respect to the control value is indicative of acute renal damage.

3. The method for the diagnosis and/or prognosis of acute renal damage according to claim 1 or 2, wherein it further comprises determining the expression level of at least one microRNA selected from miR-210 and miR-101, wherein the increase in the expression level of miR-210 and/or miR-101 with respect to the control value is indicative of acute renal damage.

4. The method for the diagnosis and/or prognosis of acute renal damage according to claim 1, wherein it further comprises determining the expression level of miR-126, miR-210 and miR-101, wherein the increase in the expression level of miR-210 and miR-101 with respect to the control value and the decrease in the expression level of miR-126 with respect to the control value is indicative of acute renal damage.

5. The method for the diagnosis and/or prognosis of acute renal damage according to any of the preceding claims, wherein microRNA expression is determined by PCR.

6. The method for the diagnosis and/or prognosis of acute renal damage according to any of the preceding claims, wherein microRNA expression is determined by quantitative PCR.

7. The method according to any of claims 1-8, wherein the expression level of microRNA is determined by RNA microarrays.

## Patentansprüche

1. Verfahren zur Diagnose und/oder Prognose einer akuten Nierenschädigung, das das Analysieren einer aus Blut oder Serum ausgewählten Probe von einem Patienten, um das Ausmaß der Expression mindestens von miR-127 zu bestimmen, und das Vergleichen des Ausmaßes der Expression mit einem Kontrollwert umfasst, wobei die Abnahme des Ausmaßes der Expression von miR-127 im Serum gegenüber dem Kontrollwert auf eine akute Nierenschädigung hindeutet.

2. Verfahren zur Diagnose und/oder Prognose einer akuten Nierenschädigung nach Anspruch 1, wobei es ferner das Bestimmen des Ausmaßes der Expression von miR-126 umfasst, wobei die Abnahme des Ausmaßes der Expression von miR-126 gegenüber dem Kontrollwert auf eine akute Nierenschädigung hindeutet.

3. Verfahren zur Diagnose und/oder Prognose einer akuten Nierenschädigung nach Anspruch 1 oder 2, wobei es ferner das Bestimmen des Ausmaßes der Expression von mindestens einer aus miR-210 und miR-101 ausgewählten Mikro-RNA umfasst, wobei die Zunahme des Ausmaßes der Expression von miR-210 und/oder miR-101 gegenüber dem Kontrollwert auf eine akute Nierenschädigung hindeutet.

4. Verfahren zur Diagnose und/oder Prognose einer akuter Nierenschädigung nach Anspruch 1, wobei es ferner das Bestimmen des Ausmaßes der Expression von miR-126, miR-210 und miR-101 umfasst, wobei die Zunahme des Ausmaßes der Expression von miR-210 und miR-101 gegenüber dem Kontrollwert und die Abnahme des Ausmaßes der Expression von miR-126 gegenüber dem Kontrollwert auf eine akute Nierenschädigung hindeutet.

5. Verfahren zur Diagnose und/oder Prognose einer akuten Nierenschädigung nach einem der vorstehenden Ansprüche, wobei die Mikro-RNA-Expression mittels PCR bestimmt wird.

6. Verfahren zur Diagnose und/oder Prognose einer akuten Nierenschädigung nach einem der vorstehenden Ansprüche, wobei die Mikro-RNA-Expression mittels quantitativer PCR bestimmt wird.

7. Verfahren nach einem der Ansprüche 1 bis 8, wobei das Ausmaßes der Expression von Mikro-RNA mittels RNA-Mikroarrays bestimmt wird.

## Revendications

1. Procédé de diagnostic et/ou de pronostic d'une lésion rénale aiguë, qui comprend l'analyse d'un échantillon provenant d'un patient choisi parmi le sang ou le sérum, la détermination du niveau d'expression d'au moins miR-127, et la comparaison dudit niveau d'expression à une valeur témoin, dans lequel la diminution du niveau d'expression de miR-127 sérique par rapport à la valeur témoin indique une lésion rénale aiguë.

2. Procédé de diagnostic et/ou de pronostic d'une lésion rénale aiguë selon la revendication 1, comprenant en outre la détermination du niveau d'expression de miR-126, dans lequel la diminution du niveau d'expression de miR-126 par rapport à la valeur témoin indique une lésion rénale aiguë.

3. Procédé de diagnostic et/ou de pronostic d'une lésion rénale aiguë selon la revendication 1 ou 2, comprenant en outre la détermination du niveau d'expression d'au moins un microARN choisi parmi miR-210 et miR-101, dans lequel l'augmentation du niveau d'expression de miR-210 et/ou miR-101 par rapport à la valeur témoin indique une lésion rénale aiguë.

4. Procédé de diagnostic et/ou de pronostic d'une lésion rénale aiguë selon la revendication 1, comprenant en outre la détermination du niveau d'expression de miR-126, miR-210 et miR-101, dans lequel l'augmentation du niveau d'expression de miR-210 et miR-101 par rapport à la valeur témoin et la diminution du niveau d'expression de miR-126 par rapport à la valeur témoin indiquent une lésion rénale aiguë.

5. Procédé de diagnostic et/ou de pronostic d'une lésion rénale aiguë selon l'une quelconque des revendications précédentes, dans lequel l'expression de microARN est déterminée par PCR.

6. Procédé de diagnostic et/ou de pronostic d'une lésion rénale aiguë selon l'une quelconque des revendications précédentes, dans lequel l'expression de microARN est déterminée par PCR quantitative.

7. Procédé selon l'une quelconque des revendications 1 à 8, dans lequel le niveau d'expression de microARN est déterminé par des microréseaux d'ARN.
